Europäisches Patentamt

European Patent Office

Office européen des brevets

(19)

(11) Publication number: **0 395 918**

**A2**

(12) **EUROPEAN PATENT APPLICATION**

(21) Application number: 90106869.2

(51) Int. Cl.⁵: **C12N 11/02, A61K 37/465**

(22) Date of filing: **10.04.90**

---

(30) Priority: **13.04.89 US 337321**
**23.03.90 US 438210**

(43) Date of publication of application:
**07.11.90 Bulletin 90/45**

(84) Designated Contracting States:
**AT BE CH DE DK ES FR GB GR IT LI LU NL SE**

(71) Applicant: VASCULAR LABORATORY, INC.
St. Elizabeth's Hospital, 736 Cambridge
Street
Boston, Massachusetts 02135(US)

(72) Inventor: **Gurewich, Victor**
**11 Reservoir Street**
**Cambridge, Massachusetts 02138(US)**
Inventor: **Breton, Jérome**
**19, Rue des Domeliers**
**F-60200 Compiègne(FR)**

(74) Representative: **Kolb, Helga, Dr. Dipl.-Chem. et**
**al**
**Hoffmann, Eitle & Partner Patentanwälte**
**Arabellastrasse 4**
**D-8000 München 81(DE)**

---

(54) Plasminogen activator complex of pure pro-urokinase covalently bound by a disulfide bridge to human serum albumin.

(57) A pro-urokinase complex consisting of pro-urokinase, covalently bound by a disulfide linkage to human serum albumin to form a fibrinolytically active plasminogen activator complex with a plasma half-life longer than that of pro-urokinase itself.

EP 0 395 918 A2

# Plasminogen Activator Complex of Pure Pro-Urokinase Covalently Bound By A Disulfide Bridge to Human Serum Albumin

## Background of the Invention

This invention relates to the use of pro-urokinase as a thrombolytic agent to lyse fibrin clots in human patients.

Pro-urokinase (pro-UK) is a single chain, 55 k dalton precursor (zymogen) of two-chain urokinase Pro-UK is described in Husain et al. U.S. Re. 32,221, hereby incorporated by reference. The activation of single chain pro-UK, by its enzymatic conversion to the two-chain urokinase form, is characteristic of the serine protease family to which it belongs. Pro-UK has been shown to have a rapid clearance time (about 5 to 8 min.) from plasma when it is injected intravenously. This clearance time is somewhat shorter than that of its active derivative, high molecular weight, two-chain UK.

## Summary of the Invention

The present invention features a plasminogen activator complex of pure pro-UK covalently bound to human serum albumin (HSA), that markedly delays the clearance of pro-UK in plasma but at the same time preserves its fibrinolytic properties. As used herein, "pro-UK" means naturally occurring or recombinant pro-UK, any pro-enzyme deletion mutant of pro-UK, or any single-chain, biologically active derivative or fragment of pro-UK which contains an available cystine residue to undergo thiol-disulfide exchange with the free cysteine of HSA, as described in greater detail below. As used herein, "HSA" means naturally occurring or recombinant HSA. As used herein, "pure" means that the complex prior to use is substantially (95% or greater by weight) free of other materials, e.g., other components of blood or tissue culture.

Because the complex of the invention has a far longer half-life in vivo than pro-UK itself, its utility in therapeutic thrombolysis is improved. In addition, the complex of the invention may be used for the prevention of thrombosis as well as the long term prevention of cardiovascular disease, such as atherosclerosis, which is well-known to be associated with a depressed fibrinolytic activity.

The pro-UK:HSA complex of the invention, because of its improved half-life in vivo, can be administered for therapeutic use by bolus intravenous injection. This represents an improvement over the therapeutic use of pro-UK itself, which, because of its short half life in vivo, has to be administered by continuous intravenous infusion, which greatly complicates therapy, especially outside a hospital.

Other features and advantages of the invention will be apparent from the following description of the preferred embodiments thereof, and from the claims.

## Description of the Preferred Embodiments

The drawings are first described.

### Drawings

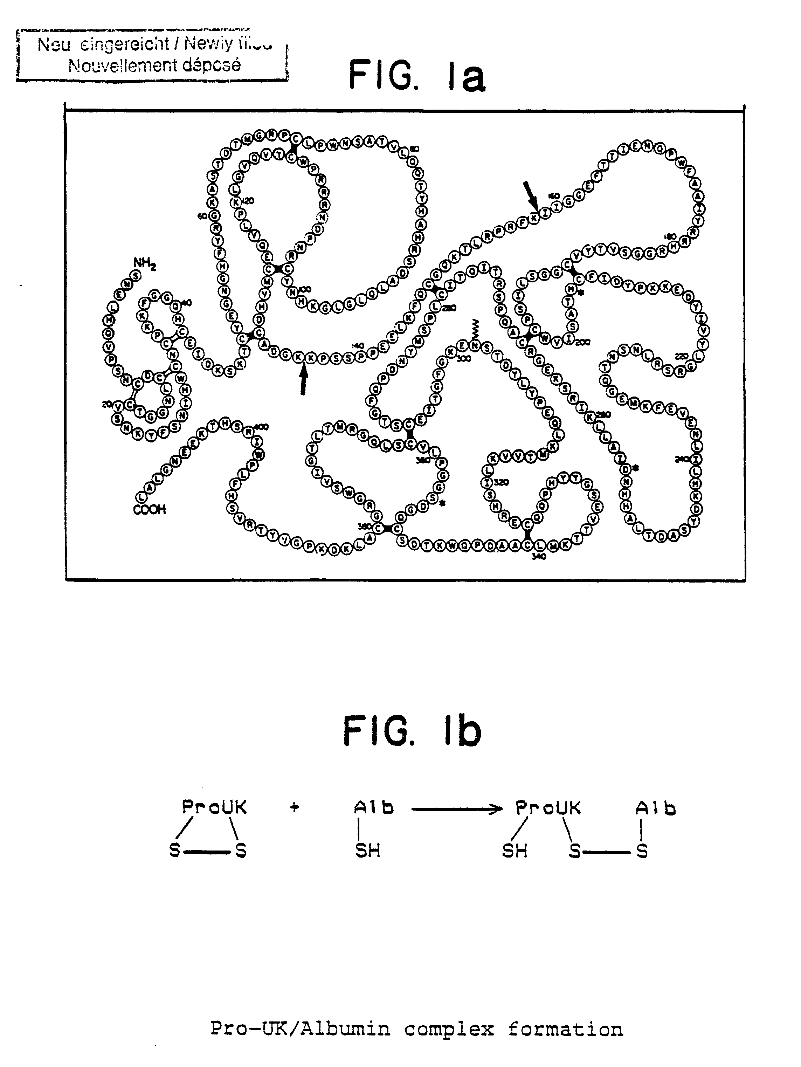

Fig. 1(a) is a schematic diagram of the full single-chain pro-UK molecule (Holmes et al., Biotechnology (1985), 3, 923-929); Fig. 1(b) is a diagrammatic illustration of single-chain pro-UK covalently bound to albumin.

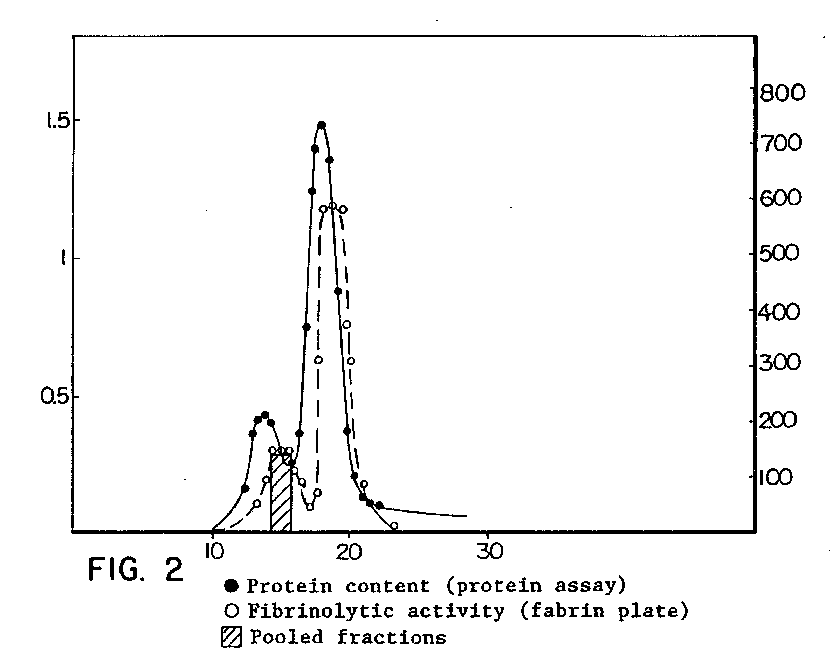

Fig. 2 shows filtration chromatography of the incubation mixture pro-UK/HSA (4h at 37°C). Fractions of 0.5 ml were collected and monitored for protein content (0) and fibrinolytic activity (0).

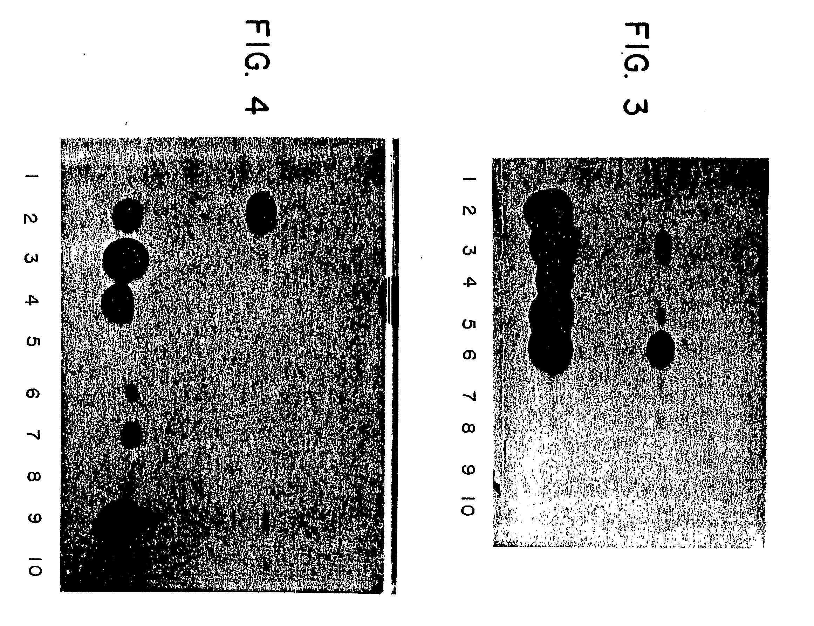

Fig. 3 is a zymogram of pro-UK incubated in buffer (lane 2), in human serum albumin (HSA) (lane 3), in CBS plasma (lane 4), in CBS plasma and HSA (lane 5), and in plasma (lane 6). Lanes 7 to 30 are pro-UK incubated with HSA after passing through an α(UK)-Sephadex column.

Fig. 4 is a zymogram of the purified complex (lanes 2,3), the purified complex incubated with α-(HSA) for 1h (lane 4), and the purified complex passed through an a(HSA)-Sephadex column. The sample in lane 3 was boiled for 2 min. in SDS buffer prior to electrophoresis instead of being treated by the standard procedure involving incubation in SDS buffer at 37°C for min.

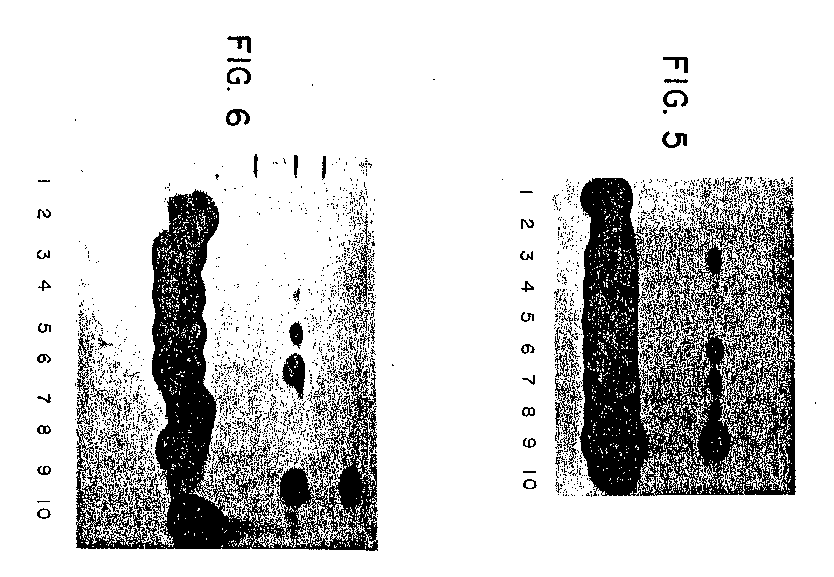

Fig 5 is a zymogram of pro-UK incubated with purified HSA at pH 5 (2), pH 9(4), and at pH 7.5 (3) with $Zn^{++}$ (6), $CA^{++}$ (7), $Zn^{++}$, $CA^{++}$ (8) and with a 10-fold higher ratio of pro-UK/HSA (9).

Fig. 6 is a zymogram of pro-UK incubated in buffer (lane 2), and in plasma for 0 (lane 3), 1h (lane 4), 2h (lane 5), and 6h (lane 6), and UK in buffer (lane 7), in plasma for 0 (lane 8) and 6h (lane 9), and in concentrated urine (lane 10).

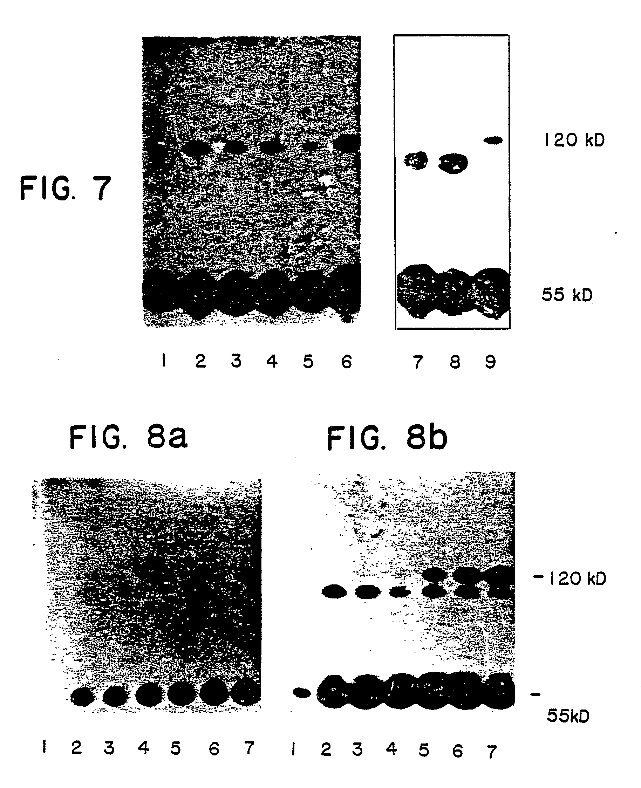

Fig. 7 is a zymogram of DFP and chloramin-T-treatment of HSA (which did not affect complexation) and DTT treatment of BSA (which restored its capacity to form complexes with pro-UK), with pro-UK in buffer (lane 1), pro-UK + HSA (lane 2), pro-UK + buffer-treated HSA (lane 3), pro-UK + DFP-treated HSA (lane 4), pro-UK + buffer-treated HSA (lane 5), pro-UK + chloramine-T-treated HSA (lane 6), pro-UK in buffer showing the presence of a dimer (lane 7), pro-UK + BSA showing only the same pro-UK dimer (lane 8) and pro-UK + DTT-treated BSA (to restore the thiol form) showing virtual elimination of the dimer and the presence of a pro-UK:BSA complex (lane 9).

Fig. 8A and 8B are zymograms showing effect of PDI on complexation-time course, with pro-UK alone in buffer (lane 1), recombinant pro-UK (0.5μg/ml) incubated with mercaptalbumin (40 mg/ml) for incubation times of 0 (lane 2), 35 min. (lane 3), 30 min. (lane 4), 1 h (lane 5), 2 h (lane 6) and 4 h (lane 7), in Fig. 8A. and the same mixtures. respectively, incubated in the presence of PDI (920 μg/ml) in Fig. 8B.

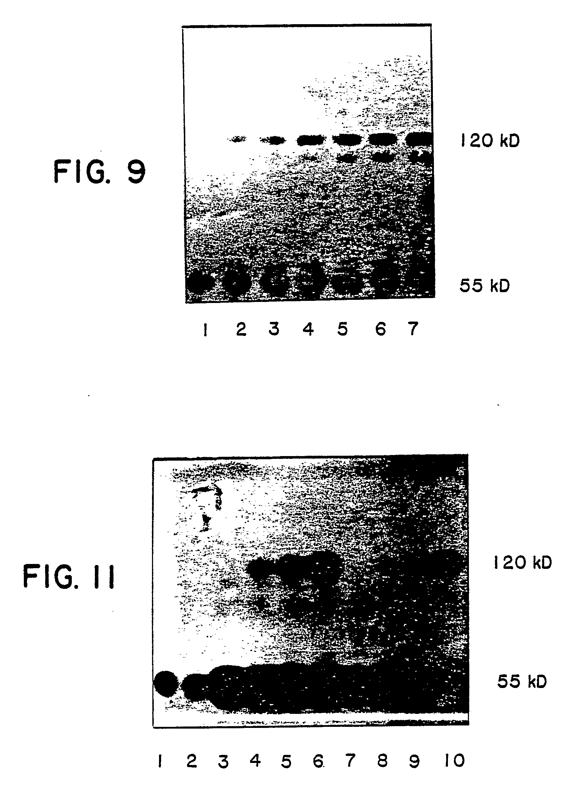

Fig. 9 is a zymogram showing the effect of PDI on complexation-dose response, with pro-UK alone in buffer (lane 1), and pro-UK (10 μg/ml) incubated with mercaptalbumin (40 mg/ml) for 6h with PDI concentrations in μg/ml of 0 (lane 2). 23 (lane 3), 230 (lane 4), 460 (lane 5), 690 (lane 6) and 920 (lane 7).

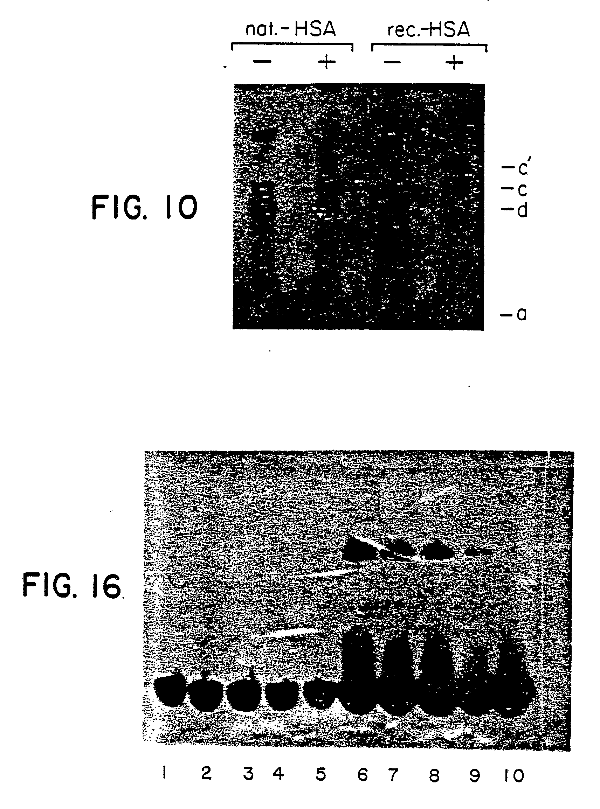

Fig. 10 is a zymogram of pro-UK complex formed with rec-HSA from E. coli.

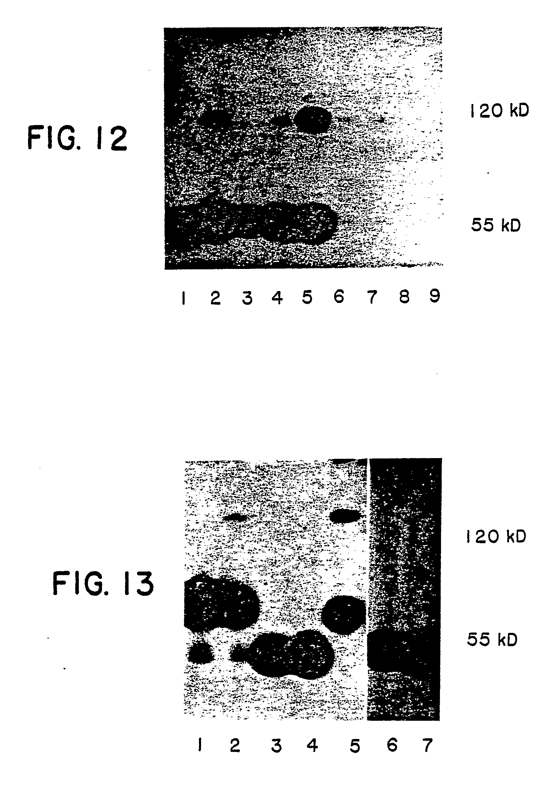

Fig. 11 is a zymogram of samples prepared in SDS at 37°C of pro-UK (0.5 μg/ml) incubated (37°C) in buffer for 0 (lane 1) and 6h (lane 2); and in plasma for 0 (lane 3), 1h (lane 4), 4h (lane 5) and 6h (lane 6); and in plasminogen-depleted plasma containing aprotinin (20 KIU/ml) for 0 (lane 7), 1h (lane 8), 4h (lane 9) and 6h (lane 10).

Fig. 12 is zymograms of the following samples: pro-UK (0.5 μg/ml) incubated (37°) in buffer (lane 1), and with CBS-HSA (40 mg/ml) (lane 2), incubated in HSA depleted plasma (lane 3), incubated in HSA depleted plasma supplemented with HSA (lane 4), plasma preincubated with pro-UK before (lane 5) and after passage over a UK-Ab Sepharose column (4 fractions are shown, lanes 6-9).

Fig. 13 is a zymogram (10% SDS-PAGE) of the following different forms of UK (0.5 μg/ml) preincubated (6h) with mercaptalbumin (40 mg/ml): HMW-UK in buffer (lane 1), HMW-UK + HSA (lane 2), LMW-UK in buffer (lane 3), LMW-UK + HSA (lane 4), rec.-pro-UK + HSA (lane 5), deletion mutant pro-UK in buffer (lane 6), deletion mutant pro-UK + HSA (lane 7).

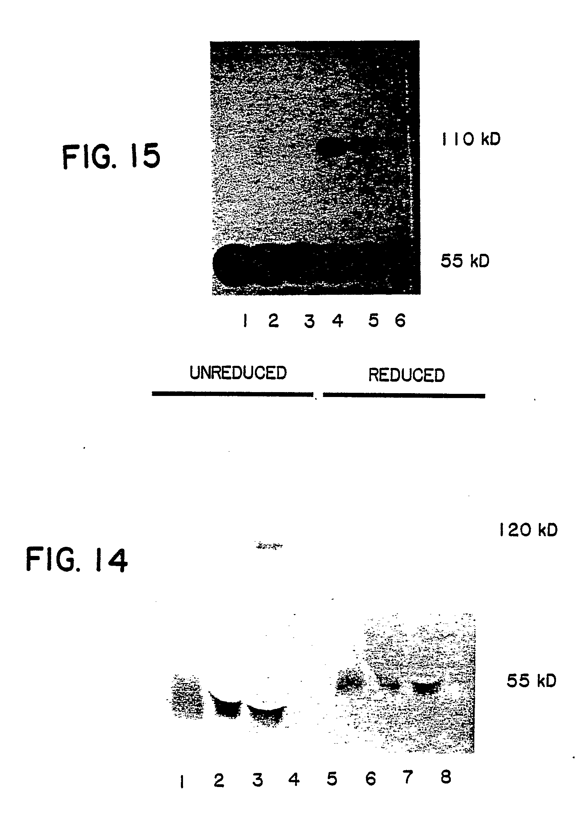

Fig. 14 is Western immunoblots using UK:Ab, pro-UK (0.3 μg/ml) incubated (37°C) 7 h in buffer (lane 1), pro-UK incubated with untreated BSA (40 μg/ml) (lane 2), pro-UK incubated with CBS-HSA (lane 3), CBS-HSA alone (lane 4), and mixtures which are the same as those in lanes 1 through 4, respectively, run under reducing conditions (lanes 5-8).

Fig. 15 is a zymogram of pro-UK incubated in buffer (lane 1) with 0.5 mM oxidized gluthathione (GSSG) (lane 2), with 5mM GSSG (lane 3), and incubated in plasma (lane 4) with 0.5 GSSG (lane 5), and with 5mM GSSG (lane 6).

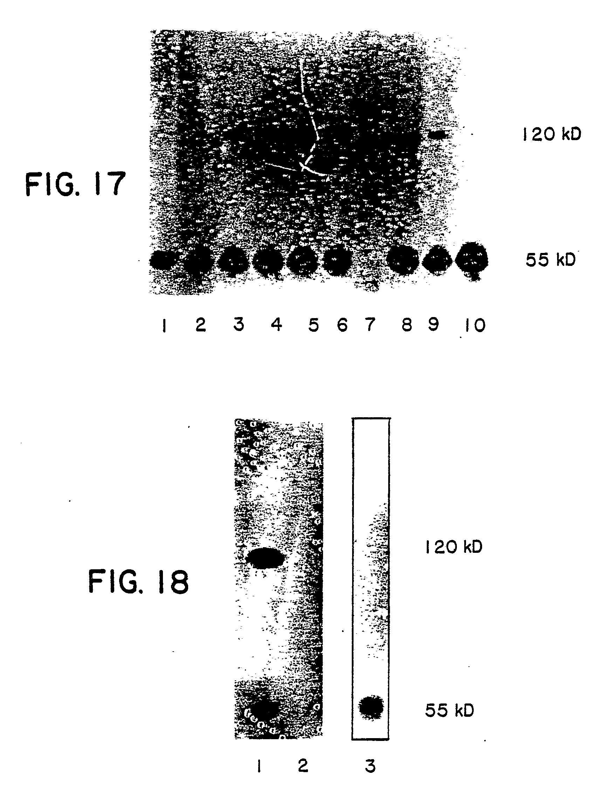

Fig. 16 is a zymogram of pro-UK incubated in buffer (lane 1) with $10^{-3}$M (2), $3.10^{-3}$M (3), $6.10^{-3}$M (4), $10^{-2}$M DTNB (5), and incubated in plasma (6) with $10^{-3}$M (7), $3.10^{-3}$M (8), $6.10^{-3}$M (9), and $10^{-3}$M DTNB (10).

Fig. 17 is zymograms showing the effect of pH and HSA concentration on complexation, with pro-UK alone in buffer (lane 1), pro-UK (5 μg/ml) incubated (37°C) for 24 h vith mercaptalbumin (40 mg/ml) at pH 6 (lane 2), at pH 7.0 (lane 3), at pH 7.4 (lane 4), at pH 8.0 (lane 5), at pH 8.5 (lane 6), and mM markers run as standards (lane 7), and Pro-UK (5 μg/ml) incubated (37°C) for 24h with HSA concentrations of 80 mg/ml (lane 8), 20 mg/ml (lane 9), and 10 mg/ml (lane 10). Lanes 8-10 incubated at pH 8.0.

Fig. 18 is zymograms of UK activity associated with CBS-HSA, with CBS-HSA from plasma preincubated (20 h) with pro-UK (0.5 μg/ml) (lane 1). CBS-HSA from plasma enriched (but not preincubated) with pro-UK (5 μg/ml) (lane 2), and UK complex immunoprecipitated from CBS-HSA isolated from fresh plasma (lane 3).

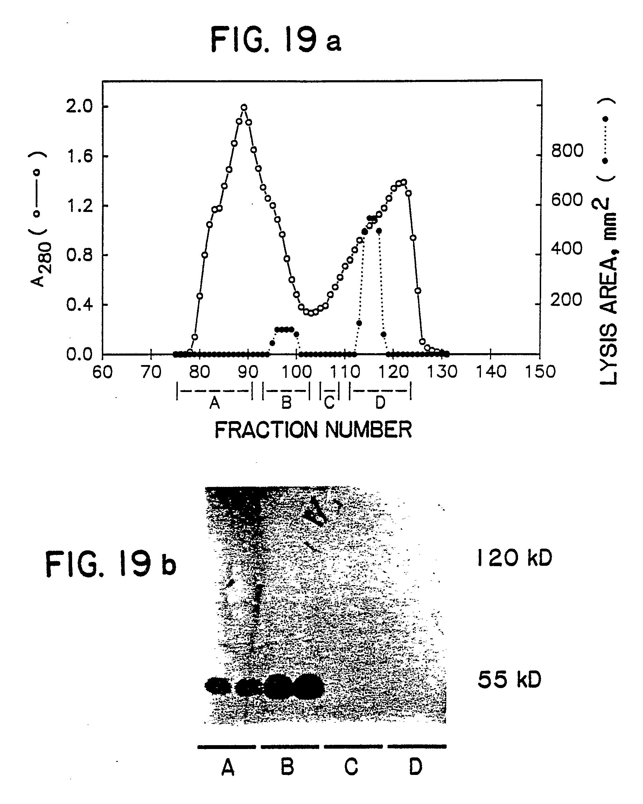

Fig. 19A is a graph of two sets of data. The fraction number of fractions collected by gel filtration of plasma on a calibrated G-75 column is plotted on the x axis for both sets of data, and the absorbance at 280nm is plotted on the y axis for data represented by open circles while the lysis area in mm² from assays on a fibrin plate is plotted on the y axis for data represented by closed circles. Fig. 193 is a zymogram of UK immonoprecipitates from the pooled fractions identified as A, B, C and D in Fig. 11A.

Characterization

The pro-UK:albumin complex of the invention exhibits the following characteristics: 1) it is more stable in sodium dodcyl sulfate (SDS) at 37°C than at 100°C; 2) it is unstable under reducing conditions; 3) complexation is promoted when the HSA pretreated with dithiothreitol (DTT) in order to restore the thiol form; 4) complexation is catalyzed by protein disulfide isomerase (PDI), and 5) complexation is inhibited by DTNB and oxidized glutathione. These characteristics indicate a disulfide linkage between a free cysteine residue in the albumin and a cystine residue in pro-UK. Based upon experiments in which complexation did not occur with either a pro-UK deletion mutant missing residues 11-135 or with LMW-UK, the cystine in pro-UK involved in a disulfide bond in the complex is believed to be in the A-chain, most likely one of the paired cystines in the NH₂-terminal end of pro-UK.

When purified HSA is stored for long periods, its capacity to form complexes with pro-UK is eventually lost due to oxidation of the free cysteine, which prevents disulfide exchange. However, its ability to form complexes with pro-UK can be restored by treatment with DTT. The latter treatment is performed at an acidic pH so that the structural disulfide bonds in the HSA are left intact.

## Methodologies

### Summary of Methodology

Two methods for producing a thrombolytic composition of the invention are described in detail below. In each method, pro-UK and albumin are first purified individually and then conjugated by means of a disulfide linkage (see Fig. 1A and 1B).

In the first method, the albumin is treated with DTT at pH 6.0 to restore the thiol form according to the method set forth in Peters, T., Advances In Protein Chemistry, 37:161-245 (1985). The DTT is then removed by dialysis prior to the conjugation of pro-UK and albumin in the presence of PDI.

In the second method, purified pro-UK and mercaptalbumin are conjugated during incubation at pH 8.0 (37° C).

### Materials and Methods

### Purification of Pro-UK

In the first method described below, pro-UK, purified from the culture medium of a human kidney cell line, was provided by Collaborative Research Inc. (Bedford, MA). To inhibit trace UK contaminants, the pro-UK was incubated with 20 μM dansyl-Glu-Gly-Arg chloromethyl ketone (GGAck) for 30 min. at 37° C in 0.1 M HEPES, 0.2 mg/ml BSA, 0.001% Tween, pH 7.4, as previously described in Pannell et al., Blood, 69: 22-26 (1987). In the second method described below, preparations of pro-UK were passed through a Sephadex G-75 column (1 x 45 cm), equilibrated and eluted with 10mM sodium acetate. 0.1 m NaCl, and 0.001% Tween, pH 4.8, in order to remove dimers of pro-UK. The pro-UK was then treated as described above to inhibit trace UK contaminants.

### Purification of Albumin

Purification of human serum albumin (HSA) from bank plasma was performed according to published procedures. A column (1.5 x 25 cm) of Cibacron Blue-Sepharose (CBS) was equilibrated with 0.63% trisodium citrate and 0.9% NaCl, and 10 ml of bank plasma was applied to the column. When the absorbance at 280 nm was less than 0.02, the HSA was eluted with 0.2 M sodium thiocyanate. dialysed against distilled water, and freeze-dried.

### Plasminogen-Free Plasma Preparation

Plasminogen-free plasma was prepared by batch separation, which was carried out by stirring 1 ml of Lys-agarose previously equilibrated in 0.005 M HEPES and 0.15 M NaCl (pH 7.4) with 5 ml plasma for 2 h at 4° C. After centrifugation, the supernatant was recovered and assayed for its plasminogen content using a standard clot lysis technique.

### Zymography and Blotting

Sodium dodecyl sulfate-polyacrylamide gel electrophoresis (SDS PAGE) was performed as described by Laemmli using a 7.5% polyacrylamide gel. Samples were prepared without reduction and heated at 37° C for 37 min. in a sample buffer (0.06 M Tris-HCl, pH 6.8, 2% SDS) prior to electrophoresis except as otherwise specified. Zymography was carried out by washing the gels with 2.5% Triton X100 and subsequently overlaying the gels on plasminogen fibrin agar plates as described by Wun et al. Western blotting was carried out based on procedures described by Towbin and Pluskal et al., with some standard modifications. The polyacrylamide gel and four filter papers were equilibrated in blotting buffer (39 mM glycine, 48 mM Tris, 20% methanol, 0.0375% SDS). The polyvinylidene difluoride (PVDF) membrane was prewetted in methanol for a few seconds and then equilibrated in distilled water for 10 min. The blotting assembly was prepared in a sandwich configuration and the electrophoretic transfer carried out with the LKB Multipore II semi-dry blotting apparatus for 1 h using a 0.8 mA/cm² current. After the transfer, the PVDF membrane was washed 10 min. with TTBS (100 mM Tris, 0.15 M NaCl, 0.1% Tween-80, pH 7.5) and quenched 1 h with 3% BSA in TTBS.

In obtaining data using the first method, immunostaining was performed after a primary incubation with rabbit anti-UK antibodies (10μg/ml) or

monoclonal Ab against HSA (1:500 dilution) in TTBS 0.25% BSA (37°C), and a secondary incubation with a 1:1000 dilution in TTBS 0.25% BSA of goat anti-rabbit or rabbit anti-mouse IgG alkaline phosphatase conjugate (Sigma) for 1 h (37°C). In obtaining data using the second method, 13 μg/ml solution of α(UK) in TTBS (with 0.25% BSA) was employed in the primary incubation (2 h at 37°C) and a 1:500 dilution in TTBS 0.25% BSA of alkaline phosphatase conjugate in the secondary incubation (2 h at 37°C).

The color reaction employed a solution of 5-bromo-4-chloro-3-indolylphosphate-toluidinic salt (0.15 mg/ml) and p-nitro blue tetrazolium chloride (0.3 mg/ml) in carbonate buffer (0.1 M sodium carbonate, 1 mM MgCl₂, pH 9.8). The PVDF membrane was washed three times for 10 min. with TTBS between each incubation. A subsequent 15 min. washing in carbonate buffer was carried out prior to the color reaction.

Enzyme Assays

Pro-UK/UK amidolytic activity was measured using standard methods with Kabi substrate S2444 at 37°C. The reaction buffer was 0.1 M Tris-HCl, 0.1 M NaCl, 0.1 mg/ml BSA, 100 u/ml aprotinin. pH 8.8; and the substrate was 0.75 mM. The reaction was stopped with saline/5% acetic acid (1:1) and the absorbance read at 405 nm. Plasminogen activator activity was measured either with standard agar fibrin plates or with Glu-plasminogen and Kabi substrate S2251.

Formation and Purification of the Pro-UK:Albumin Complex

Method 1

The thiol form of HSA or BSA was regenerated to approach the theoretical 1 SH per molecule (mercaptalbumin) by treatment with 10 mM dithiothreitol (DTT) at pH 6.0 (SD mM MES, 0.15 M NaCl) according to the method described by Peters, T., Adv. Prot. Chem., 37:161-245 (1985). Structural disulfide bonds are preserved at pH 5-7. The DTT was removed by dialysis. The pro-UK:albumin complex was prepared by incubating pro-UK (5μg/ml) in the presence of PDI (920 μg/ml) with one of the HSA preparations (40 mg/ml) or with BSA at 37°C for at least 4 h in 0.1 M HEPES buffer (pH 8.0). Subsequently, 200μl of the mixture was applied to a Sephadex G-75 column (1 x 45 cm), equilibrated and eluted with 0.1 M HEPES, pH 7.5, 0.15 M NaCl, 0.001% Tween 80, 20 KIU/ml aprotinin. Fractions of 0.5 ml were collected and monitored for protein content (absorbance at 280) and for fibrinolytic activity. The complex, under non-reducing conditions, migrates at an apparent molecular weight of ≈100 kDa, compared to standard markers. However, the complex migrates, under the conditions used, behind dimers of pro-UK. The actual molecular weight of the pro-UK:HSA complex is believed to be ≈120 kDa. Three fractions corresponding to the ≈120 kD complex were pooled.

It should be noted that immobilized PDI could be used in the incubation step instead of PDI in solution.

Method 2

The Pro-UK:HSA complex was prepared by incubating 5 μg/ml pro-UK with 40 mg/ml HSA at 37°C for 4 h in buffer 0.1 M HEPES (pH 7.4) and then applying 200 μl of the mixture to a Sephadex G-75 column equilibrated and eluted with 0.1 M HEPES, pH 7.5 0.15 M NaCl, 0.001% Tween 80, 20 KIU/ml aprotinin. Fractions of 0.5 ml were collected and monitored for protein content (standard protein assay) and fibrinolytic activity (fibrin plates) as shown in Fig. 2. (Fractions 30-31-32 were pooled.)

The pro-UK:HSA complex was recognized by an insolubilized polyclonal UK-antibody, as shown by the experiment illustrated in Fig. 3. When the purified pro-UK:HSA complex was incubated for 1 h at 37°C with an anti-HSA antibody, the 120 kDa lysis band seen by zymography was replaced by a higher molecular weight band, believed to represent the complex with HSA antibodies bound to it (Fig. 4). Similarly, the purified complex bound to an α(HSA)-Sepharose column. These findings corroborate the composition of the complex.

Preferred Conditions for Formation of the Pro-UK:Albumin Complex

The capacity of HSA or BSA to form a complex with pro-UK decreases when the HSA or BSA is stored for long periods of time, due to oxidation of the free cysteine. Testing of commercial HSA, which was not fresh, by 5,5´-dithiobis(2-nitrobenzoate) (DTNB) titration of available free cysteine (under native conditions) revealed 1 free cysteine per about 10 molecules of commercial HSA. In contrast, HSA freshly purified as described above in method 2 contained 1 free cysteine per about 2 molecules of HSA. Therefore, DTT treatment [pH 6.0] of HSA or BSA is recommended before incubation for complex formation

A pH of about 8.0 is preferred for complex formation; as shown in Fig. 5. The pro-UK:HSA complex formation occurred at pH 7.5 (lane 3), but not at pH 5 (lane 2) or pH 9 (lane 4). The addition of $Ca^{++}$ (5 mM) (Fig. 5, lane 7) or $Zn^{++}$ (50 $\mu$M) (lane 6) and $Ca^{++}$ + $Zn^{++}$ (lane 8) had no effect, but $Cu^{++}$ (5-10 $\mu$M) inhibited complex formation.

Experimental Data

Spontaneous Formation of Pro-UK:Albumin Complex in Plasma

The pro-UK:HSA complex was found to form spontaneously in normal plasma, and HSA purified from normal plasma was shown to be associated with pro-UK. Therefore the pro-UK:HSA complex is believed to exist in nature. In these experiments, pro-UK was incubated with plasminogen-free plasma, and the molecular weight of the plasminogen activator activity determined by zymography.

Pro-UK was added to plasma (500 ng/ml) and incubated at 37°C for one to six hours. In time, a portion of the enzymatic activity migrated at an apparent molecular weight of about 100 kDa (actual molecular weight about 120 kDA), as shown in the zymogram of Fig. 6. This zymogram shows pro-UK (lanes 2-6) and (8-9) in buffer and incubated in plasma for 0 (lanes 3, 8), 1 (4), 2 (5), or 6 hours (6, 9). Generation of this new high molecular weight (HMW) band in plasma was a slow and time-dependent phenomenon. About 10% of the enzymatic activity was found at ≈120 kDa (assumed actual) after 6 hours of incubation in plasma, though quantitation by zymography is at best a crude estimate. Pro-UK dimer formation was excluded as demonstrated by the absence of a HMW lysis band when pro-UK was incubated in buffer. Moreover when pro-UK dimers did form, they migrated ahead of the complex (See Figs. 7, 8B, 9, 10). The findings indicate that pro-UK binds to an ≈65k dalton plasma protein to form an ≈120 k dalton compound.

A complex of similar MW made up of two-chain UK and a UK inhibitor, PAI-3, has been observed previously in both urine and plasma. However, this complex can be excluded as an explanation for the above findings, for the following reasons: (1) Pro-UK does not form a complex with PAI-3, and (2) in plasma pro-UK is stable and is not activated to two-chain UK at the concentration used in the experiments. Finally, the complex formation was not prevented by addition of an inhibitor to (GluGlyArg chloromethyl ketone), an inhibitor to plasminogen (aprotinin 20 KIU/ml) or by making the plasma plasminogen-free.

Moreover, activation of pro-a during incubation and formation of a UK: PAI-1 (plasminogen activator inhibitor-1) complex does not fit the findings since the latter migrates at ≈95 kD and is accompanied simultaneously by other inhibitor complexes at ≈125 kD and ≈155 kD (Kruithof, E.K.O. et al; Blood, 64:907-913 (1984)), as well as a fourth complex at the top of the gel which reacts with $\alpha_2$-macroglobulin-Ab. Nevertheless, to reduce the possibility of pro-UK activation during incubation, additional experiments were conducted in which aprotinin (20 KIU/ml) was added to plasma depleted of plasminogen. These procedures did not inhibit the formation of the ≈120 kD complex (Fig. 11, lanes 7-10).

However, no high molecular weight activator complex was generated when pro-UK was incubated with HSA-depleted plasma (CBS plasma), as shown in Fig. 3. These findings demonstrate that the complex observed in plasma is composed of pro-UK complexed with HSA.

Generation of a Pro-UK Complex with CBS-HSA

When pro-UK (5$\mu$g/ml) was incubated (37°C) for at least 4 h with freshly purified CBS-HSA (40 mg/ml in 0.1 M HEPES, pH 7.4), and the mixture examined by SDS-PAGE and zymography, an ≈120 kD band consistently formed (Fig. 12, lane 2). By contrast, when pro-UK was incubated in CBS-depleted plasma, no complex formed (Fig. 12, lane 3). Repletion of the CBS-depleted plasma with CBS-HSA restored complex formation (Fig. 2, lane 4). Passage of plasma containing the complex (Fig. 2, lane 5) over a UK-Ab Sepharose column removed the complex (Fig. 2, lanes 6 - 9).

Complex formation was also observed when pro-UK was incubated with commercial preparations of HSA and BSA, provided that they were pretreated with DTT in order to restore the thiol form.

Sensitivity of the Complex to Dissociation in SDS

The complex which formed after incubation of the pro-UK with CBS-HSA was isolated by gel filtration on Sephadex G-75 and then analyzed by SDS-PAGE. When the sample was boiled, complete dissociation of the complex occurred, whereas at 37°C only partial (<50%) dissociation was seen on zymograms of the gel (data not shown).

Complexation by Different Forms of UK with HSA and the Effects of DFP and Chloramine-T on Complexation

The possibility that the complex was actually composed of UK and an inhibitor co-purified with HSA on the CBS column was investigated since it was found that HMW-UK also formed a complex when incubated with HSA (Fig. 13, lane 2) which was not seen when HMW-UK was incubated in buffer (Lane 1). The complex migrated at ≈120 kD corresponding to the complex formed when pro-UK was incubated in plasma (Fig. 13, lane 5). However, incubation of LMW-UK (Abbokinase) with HSA failed to induce a complex (Fig. 13, lanes 3 & 4). Moreover, no complex formed when a deletion mutant of pro-UK (missing residues 11-135) was incubated with HSA (Fig. 13, lane 7). These observations indicated that the complex was not with an inhibitor of UK and that complexation involved the A-chain of UK and· not the protease moiety of the B-chain.

To further exclude a UK:inhibitor complex, the CBS-HSA was pretreated with DFP in order to inhibit any serine protease contaminants. This treatment failed to inhibit complex formation with pro-UK (Fig. 7, lane 4, compared with lanes 2 & 3). Second, to exclude a serpin contaminant, the HSA preparation was treated with chloramine-T, shown to inactivate serpins (Stief et al., Biol. Chem. Hoppe-Seyler, 369:1337-1348 (1988) ). This treatment also failed to inhibit complexation establishing that the observed pro-UK or HMW UK complex formed was not with an inhibitor (Fig. 7, lane 6 compared with the untreated HSA control, lane 5).


Evidence that the Complex is Disulfide Linked

Western immunoblotting using a polyclonal UK-Ab showed a ≈120 kD band when pro-UK was incubated with CBS-HSA (Fig. 14, lane 3), but not when pro-UK was incubated in buffer (Fig. 14. lane 1) or with BSA that had not been pretreated with DTT (Fig. 5, lane 2). The CBS-HSA control did not give a visible reaction with the antibody (Fig. 14, lanes 4 & 8). The ≈120 kD complex dissociated under reducing conditions, suggesting that it was disulfide-linked (Fig. 14, lane 7).

Additional evidence indicates that the pro-UK:albumin complex is disulfide-linked. First, complexation was inhibited when a sulfhydryl reagent like oxidized glutathione or DTNB was added to the reaction mixture of method 2, as follows. Oxidized glutathione was added to plasma and allowed to react for 2 h at 37°C. The results are shown on the zymogram in Fig. 15. Lanes 1-3 of the zymogram show pro-UK incubated in buffer. A decrease in pro-UK:HSA complex formation occurred with glutathione which was concentration dependent. Lanes 2 and 5 are with 0.5 mM added glutathione and lanes 3 and 6 with 5.0 mM added glutathione.

Glutathione did not inhibit the fibrinolytic activity of pro-UK, as shown in the first three lanes. This response to glutathione indicates that the free cysteine of the albumin is involved in the complex, and that therefore pro-UK and HSA are linked by a disulfide bridge. Similarly, experiments with DTNB, which reacts with a free cysteine. showed that DTNB inhibited complex formation. Fig. 16 is a zymogram of pro-UK incubated in buffer (lanes 1-5) and with HSA (lanes 6-10) with 0.0 M (lanes 1, 6), 1 mM (lanes 2, 7), 3 mM (lanes 3, 8), 6 mM (lanes 4, 9), and 10 mM DTNB (lanes 5, 10).

Second, commercial preparations of HSA or BSA formed little or no complexes with pro-UK. This is illustrated in (Figure 7, lane 8). which shows only a pro-UK dimer, and in (Figure 14, lane 2). Furthermore, complexation with freshly prepared CBS-HSA decreased with storage time. However, the capacity to form complexes with pro-UK by all of these preparations could be restored when they were treated with DTT. This effect on a commercial BSA preparation is illustrated in (Figure 7, lane 9). The effect of DTT treatment at pH 5-7 is to restore the thiol form without dissociating structural disulfide bonds, as described by Peters, T. Adv. Prot. Chem., 37:161-245 (1985). Therefore, the findings indicate that an available cysteine thiol in the HSA or BSA is necessary for the complexation with pro-UK.

Third, titration with DTNB revealed that before DTT treatment, commercial HSA had 0.1 available cysteines compared with 0.6 after treatment. A comparable figure was obtained with freshly prepared CBS-HSA. There was, therefore, a positive correlation between available cysteines in the HSA preparation and complexation with pro-UK.

Fourth, when PDI, which catalyzes thiol disulfide exchange (Freedman, R.B., Cell, 57:1069-1072 (1989); Gilbert, H.F., Biochem., 28:7298-7305 (1989)) was included in the incubation mixture, complexation was accelerated. Without PDI, 4 h of incubation were required before complexes were seen (Fig. 8A), but in the presence of PDI (920 μg/ml) complexation occurred after 1 h incubation (Fig. 8B). The PDI effect was concentration-dependent as illustrated in Fig. 9 in which pro-UK (10μug/ml) was incubated for 6 h with a mercaptalbumin preparation (40 mg/ml) with 0, 23, 230, 460, 680 or 920 μg/ml of PDI. In the presence of PDI, pro-UK dimer formation was also much enhanced (Figs. 8A, 8B, and 9) suggesting that these dimers are disulfide-linked. The large amount of PDI required is consistent with the low efficiency of this enzyme.

Complexation was inhibited when the sample was boiled for 2 min. in SDS (Fig. 14, lane 3). This finding is also consistent with a disulfide-linked complex which may be destabilized by boiling.

Moreover, in the presence of $CuCl_2$ (1 mM). which inhibits disulfide exchange, the complex resisted boiling. The thermal dissociation of disulfide linkages has been previously reported by Volkin and Klibanov for certain disulfide-linked conjugates (Volkin et al., J. Biol. Chem., 262:2945-2950 (1987)).

Disulfide linkage between a cystine of pro-UK and an unpaired cysteine of albumin would create a free cysteine in the albumin-linked pro-UK. This free cysteine could react with a cystine in another pro-UK molecule, or a cysteine in another albumin molecule. Under such circumstances, instead of a complex made up of one molecule of pro-UK and one molecule of albumin, a complex would be formed of either two pro-UK molecules linked to one albumin molecule, or two albumin molecules linked to one pro-UK molecule. These tripartite complexes, have been identified and are within the scope of the present invention.

Effect of pH and Concentration of HSA

When incubation was carried out at a pH range from 6-8.5, the optimal pH for complexation appeared to be about pH 8-8.5 (Fig. 17, lanes 1-6). Most experiments were therefore carried out at pH 8.0. Experiments with a wide range of ratios of the HSA preparations to pro-UK revealed that the predominant limiting factor determining complexation was the concentration of the HSA. This is illustrated in Fig. 17 (lanes 8-10) in which 80, 20 and 10 mg/ml of DTT-treated commercial HSA were incubated with pro-UK (5μg/ml). Raising the concentration of the pro-UK did not enhance complexation. Moreover, complexation even with this excess of HSA tended to reach a plateau by about 24 h.

The Effect of Further Purification of the HSA

The above findings suggested the possibility that the pro-UK complex was with a minor contaminant present in the HSA as well as in the BSA preparations. Attempts to remove such a contaminant by chromatography of the HSA preparations with DEAE cellulose or passage over concanavalin-A Sepharose did not alter the reactivity of the preparations to pro-UK. Therefore, if a minor protein contaminant was responsible, it was unlikely to be with a non-glycoprotein. With few exceptions (HSA. transthyretin, retinol binding protein), plasma proteins are all glycoproteins (Peters, T., Adv. Prot. Chem., 37:161-245 (1985)).

Antibody Recognition of the Complex

The pro-UK complex which was formed with highly purified, DTT-treated HSA according to method 1 was electro-eluted from gel sections after SDS-PAGE, and then again subjected to SDS-PAGE. Immunoblotting with UK-Ab revealed the complex at ≈120 kD. However, although the complex was shown to be recognized by polyclonal HSA-Ab in numerous experiments, it was not recognized by a monoclonal, HSA-Ab in this experiment (data not shown). The explanation for this discrepancy may be related to the essential epitope on the HSA recognized by the monoclonal antibody being masked by the complex. Nevertheless, this negative finding left open the possibility that the pro-UK complex was with a minor plasma protein contaminant of HSA or BSA. Therefore, studies were conducted with rec-HSA from E. coli, free of all plasma protein contaminants.

Complexation of Pro-UK with rec-HSA

Rec-HSA and native HSA (40 mg/ml), before (-) and after (+) treatment with DTT, were each incubated (6h) with pro-UK (5 μg/ml). Zymographic analysis of the incubation mixtures showed a ≈120 kD complex (c) with both native and rec-HSA whose formation was significantly enhanced by DTT pretreatment (+) of the HSA preparations. The DTT treatment also promoted the formation of higher molecular weight complexes (c′) possibly involving the unpaired cysteine in the pro-UK complexed with HSA. Dimers (d) of pro-UK were seen migrating just ahead of the ≈120 kD complex (Fig. 10).

Separation of the Pro-UK Complex from Plasma by CBS Affinity Chromatography

It was determined that uncomplexed pro-UK was not copurified with HSA by CBS affinity chromatography after extensive washing with 0.5 M NaCl. This was shown by experiments in which pro-UK (5 μg/ml) was added to plasma immediately prior to CBS affinity chromatography. Under these conditions, the eluted HSA had no detectable fibrinolytic activity on a zymogram (Fig. 18, lane 2). The free pro-UK is gradually washed off with 0.5 M NaCl. By contrast, when plasma was preincubated (37° C) with pro-UK (5 μg/ml) for 20 h, in order to permit complex formation, a zymogram of the CBS-purified HSA had fibrinolytic activity associated with it. This was shown by zymography of the HSA which showed bands migrating at both ≈ 120 kD and ≈ 55 kD (Fig. 18, lane 1). Since free pro-UK was shown not to be copurified, the ≈ 55 band presumably represented some pro-UK which dissociated from the HSA-complex during sample preparations in SDS.

Studies of Pro-UK Intrinsic to Plasma

To determine if pro-UK intrinsic to plasma might be in a similar complex with HSA, approximately 600 mg of HSA was purified from fresh plasma by CBS chromatography with extensive washing of the column with 0.5 M NaCl. This preparation was treated with Staphylococcal protein A anti-UK antibody adsorbent previously shown (Fig. 12, lanes 6-9) to recognize the pro-UK in the complex. The immunoprecipitate was boiled in SDS sample buffer in order to dissociate the antigen antibody complex. These conditions also cause the pro-UK:HSA complex to dissociate. Subsequent zymography revealed a ≈ 55 kD lysis band indicating the presence of pro-UK in the HSA preparation (Fig. 18 lane 3). Since free pro-UK was shown not to be copurified by CBS affinity chromatography, the findings suggest that the pro-UK isolated from plasma on the CBS column had been in a complex with HSA. Therefore, the findings in normal plasma paralleled those observed when pro-UK-enriched plasma was incubated for ≥6 h. The possibility that the UK isolated by CBS affinity chromatography was a UK:inhibitor complex, rather than a pro-UK:HSA complex, was considered unlikely since UK forms inhibitor conjugates which are stable in SDS at 100°C (Kruithof, Blood, 64:907-913 (1984); Cieplak et al., Thrombos. Haemostas., 53:36-44 (1985); Stump et al., J. Biol. Chem., 261: 12834-12841 (1986)), and which therefore migrate on SDS-PAGE at )95 kD.

In order to investigate the proportion of the pro-UK intrinsic to plasma which is present as a complex, 20 ml of fresh plasma (containing 20 KIU/ml aprotinin) was chromatographed on a Sephadex G-75 column. This resolved plasma proteins into two main peaks (Fig. 19A). The positions of free pro-UK and of the pro-UK:HSA complex were determined by calibrating the column with plasma samples enriched with pro-UK just before gel filtration and with plasma samples preincubated (37°C for 20 h) with pro-UK (5 μg/ml) so as to form the complex. The fibrinolytic activity in the collected fractions was assayed on fibrin plates. The free pro-UK was found in fractions 112-119 corresponding to pool D, and the pro-UK:HSA complex was found in fractions 95-100 corresponding to pool B (Fig. 19A).

Thereafter, normal plasma was analyzed by gel filtration on this column. The fibrinolytic activity intrinsic to the plasma was assayed by immunoprecipitation and zymography. The fractions were pooled into 4 lots as shown in Fig. 19A. As shown in Fig. 19B, fibrinolytic activity was detectable predominantly in pool B which corresponded to the high molecular weight fractions in which the pro-UK complex was found to have been eluted (Fig. 19A). A small amount of UK activity was found at an even higher MW (pool A) (Fig. 19B). Higher molecular weight pro-UK:HSA complexes were also formed in some of the experiments in which pro-UK was incubated with purified HSA (see Fig. 10), presumably representing complexes with more than one molecule of HSA or pro-UK. No UK activity was found in the pooled fraction D corresponding to the MW of free pro-UK. Therefore, all the detectable UK activity in plasma was found to be present in the form of a complex. Since the complex dissociated in SDS during sample preparation as shown by the ≈ 55 kD band on the zymogram (Fig. 19B), the observed complex is inconsistent with a UK:inhibitor complex. The findings therefore suggest that most of the pro-UK intrinsic to normal plasma is in a complex corresponding to the complex which forms with HSA.

The Long Half-Life of the Complex in the Circulation

It is postulated that the site on the pro-UK molecule identified by the UK receptor in the liver is masked in the complex of the invention and as a result, the bound pro-UK is provided with a long (several hours to several days) half-life, in contrast with the 5-8 minute half-life of free pro-UK. Since the fibrinolytic properties of pro-UK are not compromised, the pro-UK:albumin complex is expected to be well suited for therapeutic thrombolysis. The invention should substantially reduce the dosage of pro-UK required, and facilitate administration by eliminating the need for a constant infusion.

Use

The pro-UK:albumin complex of the invention can be used therapeutically for the same indications as other plasminogen activators, e.g., t-PA, UK, and pro-UK. The purpose is to dissolve intravascular blood clots (thrombi). Current clinical indications include myocardial infarction, deep vein thrombosis, and pulmonary embolism. The complex of the invention is mixed with a biologically suitable carrier substance, e.g., saline, and administered intravenously by bolus injection. The complex also may be advantageously mixed with t-PA, with which pro-UK is synergistic. This mixture can also be given as a simple bolus injection which simplifies treatment and enables it to be carried out at home. The t-pA will initiate lysis and be cleared rapidly while the long-acting pro-UK will complete the lytic process.

Additionally, the complex of the invention can be administered for the purpose of raising the intrinsic fibrinolytic activity of blood and thereby used for the long term prevention of cardiovascular disease known to be associated with a depressed fibrinolytic activity. Since thrombosis has long been believed to be part of the pathogenesis of atherosclerosis, regular injections of the complex of the invention at weekly or monthly intervals may be administered to prevent atherosclerosis as well.

Finally, the enzymatically active form of the complex, between HMW-UK and albumin, may also have certain clinical applications. Since HMW-UK is the enzymatic form of pro-UK, it reacts with several plasma inhibitors. As a result, this complex will not have a long half-life until these inhibitors have been consumed. Thereafter, the complex will have the advantage over HMW or LMW-UK of a longer half-life which will reduce cost and facilitate administration.

Examples of Therapeutic Regimes

Example 1

For emergency treatment of thrombi by bolus injection, about 5-20 mg of the lyophilized pro-UK:albumin complex is mixed with saline and placed into the chamber of a syringe, which is used to inject the bolus into the patient intravenously.

Example 2

For the rapid lysis of coronary thrombi, about 5-20 mg of the lyophilized pro-UK:albumin complex dissolved in saline is administered intravenously together with 2 mg UK. The latter serves to accelerate the initiation of lysis. The long half-life of the complex should serve to prevent rethrombosis as well as to complete the lysis of existing thrombi.

Example 3

For the treatment of deep vein thrombi and pulmonary emboli, about 5-20 mg of the lyophilized pro-UK:albumin complex dissolved in saline is administered intravenously. Injections can be repeated if necessary. Non-specific plasminogen activation should not occur at these doses.

Example 4

For treatment of thrombi by bolus injection of synergistic combinations of t-PA and the lyophilized pro-UK:albumin complex, about 5-20 mg of t-PA is mixed with 5-20 mg pro-UK:albumin complex and administered as a simple bolus injection. The t-PA will be cleared rapidly but the complex will remain in the circulation to complete lysis of thrombi and prevent rethrombosis without compromising specificity.

Example 5

Alternatively, for the rapid lysis of coronary thrombi, about 5-20 mg of lyophilized pro-UK:albumin complex is injected together with an infusion of about 30 mg/hr of lyophilized t-PA dissolved in saline.

Example 6

The exceptionally long half-life of the pro-UK:albumin complex may make it useful for the prevention of thrombosis. This application would utilize small doses, i.e. about 1-5 mg weekly.

Example 7

The active HMW-UK:albumin complex may be used as a more economical substituion for HMW or LMW-UK.

Example 8:

For the treatment of deep vein thrombi, about 5-20 mg of the lyopholized pro-UK:HSA complex is dissolved in saline and administered either alone or in combination with t-PA.

Example 9:

For the treatment of unstable angina or impending myocardial infarction, a bolus of the pro-UK:HSA complex (5-10 mg) is injected intravenously. For this indication, a plasminogen activator with a long half-life is particularly useful since this clinical condition tends to last for several days.

## Example 10:

For the prevention of rethrombosis, following percutaneous transluminal angioplasty or following acute thrombolytic therapy with another plasminogen activator, a relatively small dose of the pro-UK:HSA complex of the invention should be sufficient, i.e. less than 10 mg.

## Example 11

For the prevention of cardiovascular disease which is often associated with a depressed fibrinolytic activity, periodic injections of the complex may be given to increase fibrinolytic activity. For example, monthly injections of about 1-5 mg of pro-UK in complex with HSA may be given.

## Claims

1. A purified plasminogen activator complex comprising pure pro-urokinase covalently bound to human serum albumin through a disulfide linkage between a sulfur atom in an amino acid residue of said pro-UK and sulfur atom in an amino acid residue of said human serum albumin.

2. The activator complex of claim 1, wherein said disulfide linkage is between a cysteine residue of said human serum albumin and a cystine residue of said pro-UK.

3. The activator complex of claim 1 wherein said cystine residue of said pro-UK is in the amino terminal end of pro-UK in the A chain.

4. The activator complex of claim 1 wherein the formation of said disulfide linkage takes place in the presence of protein disulfide isomerase.

5. The activator complex of claim 1, said complex having the same molecular structure as the complex of pro-UK and human serum albumin which exists in the blood of healthy human individuals.

6. The activator complex of claim 2 wherein said disulfide linkage is between the same cysteine residue of human serum albumin and the same cystine residue of pro-UK as in the complex of pro-UK and HSA which exists in the blood of healthy human individuals.

7. The activator complex of claim 1 or 6 wherein said pro-UK is recombinantly produced pro-UK.

8. The activator complex of claim 1 wherein said pro-UK and said human serum albumin are recombinantly produced.

9. The activator complex of claim 1 wherein said pro-UK is a fibrinolytically active fragment of pro-UK having a cysteine residue in its amino terminal end at any one of the amino acid positions 11, 31, 33, 39, 51, 53, or 63 of the pro-UK peptide chain.

10. The activator complex of claim 1 wherein said pro-UK is a fibrinolytically active zymogen form of urokinase containing an amino acid chain identical to amino acid residues 1 through 45 of urokinase.

11. A therapeutic composition comprising the activator complex of claim 1, admixed with a pharmaceutically acceptable carrier substance.

12. A method of producing the thrombolytic activator complex of claim 1 comprising incubating pure human serum albumin and pure pro-urokinase under conditions and for a time sufficient to allow the formation of said complex.

13. The method of claim 12 further comprising pretreating the human serum albumin with dithiothreitol to restore the thiol form of human serum albumin.

14. The method of claim 12 or 13 further comprising adding protein disulfide isomerase during said incubation.

15. The use of the therapeutic composition of claim 11 for the preparation of a pharmaceutical.

16. The use of the therapeutic composition of claim 11 for the preparation of a thrombolytic pharmaceutical.

17. The use of the composition of claim 11 for the preparation of a pharmaceutical useful for treating or preventing cardiovascular diseases, such as arteriosclerosis.

Neu eingereicht / Newly filed
Nouvellement déposé

# FIG. la

# FIG. lb

Pro-UK/Albumin complex formation

FIG. 2

● Protein content (protein assay)
○ Fibrinolytic activity (fabrin plate)
▨ Pooled fractions

EP 0 395 918 A2

FIG. 3

1  2  3  4  5  6  7  8  9  10

FIG. 4

1  2  3  4  5  6  7  8  9  10

FIG. 5

1 2 3 4 5 6 7 8 9 10

FIG. 6

1 2 3 4 5 6 7 8 9 10

FIG. 7

120 kD

55 kD

1  2  3  4  5  6      7  8  9

FIG. 8a        FIG. 8b

- 120 kD

- 120 kD

-

1 2 3 4 5 6 7     1 2 3 4 5 6 7

55kD

FIG. 9

120 kD

55 kD

1  2  3  4  5  6  7

FIG. 11

120 kD

55 kD

1  2  3  4  5  6  7  8  9  10

nat.-HSA        rec.-HSA
  −     +        −     +

FIG. 10

−c′
−c
−d

−a

FIG. 16

1  2  3  4  5  6  7  8  9  10

FIG. 12

120 kD

55 kD

1 2 3 4 5 6 7 8 9

FIG. 13

120 kD

55 kD

1 2 3 4 5 6 7

FIG. 15

110 kD

55 kD

1  2   3  4   5  6

UNREDUCED          REDUCED

120 kD

FIG. 14

55 kD

1    2    3    4    5    6    7    8

FIG. 17

120 kD

55 kD

1  2  3  4  5  6  7  8  9  10

FIG. 18

120 kD

55 kD

1   2   3

# FIG. 19 a

# FIG. 19 b

120 kD

55 kD

A    B    C    D